# EUROPEAN PATENT SPECIFICATION

(11) **EP 3 938 106 B1**
(45) Date of publication and mention of the grant of the patent: **17.01.2024**
(21) Application number: 20715126.7
(22) Date of filing: 13.03.2020
(51) Int. Cl.: B01L 3/00, B01L 9/06, C12M 1/32

(54) **DIVISIBLE MULTI-WELL PLATES**
TEILBARE PLATTE MIT MEHREREN VERTIEFUNGEN
PLAQUES MULTIPUITS DIVISIBLES

(30) Priority: 14.03.2019 US 201962818669 P
(43) Date of publication of application: 19.01.2022
(73) Proprietor: Abgene Limited, Altrincham Cheshire WA14 2DT (GB)
(72) Inventor: SIMS, Daniel, Ashford Kent TN23 4FD (GB); PRYCE, Peter, Ashford Kent TN23 4FD (GB); NAGY, Gabor, Ashford Kent TN23 4FD (GB)
(74) Representative: HGF
(86) International application number: PCT/GB2020/050647
(87) International publication number: WO 2020/183195

(56) References cited:
- US-A- 4 875 620
- US-A1- 2013 029 343

## Description

### TECHNICAL FIELD

The present disclosure relates broadly, but not exclusively, to multi-well plates that can be manually divided into strips. Manually divided tube strips of the disclosure have smooth edges that prevent injury or glove tearing to a user.

### BACKGROUND

Reagents for biological and chemical methods are often supplied to users, pre-aliquoted in multi-well plates, such as 96-well plates. Often these reagents are supplied as lyophilized and/or frozen formulations. In a typical multi-well plate configuration, for a frozen reagent, a user would need to thaw the entire multi-well plate and either use all the reagents at once or discard unused sections of the plate. Reagents can alternatively be supplied pre-aliquoted in strips of tubes (such as 8-tube strips), but this is not suited to the manufacturing environment because strip tubes need to be manually loaded into automated filling equipment and manually sealed with cap strips. Multi-well plates on the other hand have been standardized for automatic reagent dispensing and sealing.

Some existing multi-well plates are divisible into tube strips or segmented plates. However, these are manufactured entirely from polypropylene or similar soft material, which, while suitable for reactions that require temperature changes such as the polymerase chain reaction (PCR) are not suitable for automation systems and manufacturing processes. Due to characteristics of the material, these tube strips or segmented plates are hard to handle by humans or automation systems in a manufacturing environment. For example, the softness of polypropylene makes the plates difficult to grip by automated handlers and robotic devices. Grippers used by automation systems could warp, deform or crush the plate due the flexible, semi-rigid nature of polypropylene material.

Some existing polypropylene divisible plates require scissors, cutters or other cutting tools to be divided into sections.

In addition, some existing divisible multi-well plates are only divisible into segmented plates, i.e., are divisible into 24-well or 32-well segments and not into single 8-tube strip. This again results in wastage of unused reagents.

There are some divisible multi-well plates that have a frame made of a firm material and wells made of a soft material that are suitable for automated handling. However, in some cases, such multi-well plates require a tool such as a scissor or a cutter, for breaking the multi-well plate apart into tube strips. In other cases, such multi-well plates, are manually divisible but they are only divisible into segments (such as 32 well blocks) and are not amenable for dividing into single strips (such as either tube strips). In yet other cases, in existing manually divisible multi-well plates, which can be divided into single strips, the dividing process leaves sharp edges that cause glove tears or injury to users.

A need therefore exists for multi-well plates that are manually divisible, without use of any cutting tool, in single tube strip formats that that address at least some of the problems described above.

US 2013/029343 relates to a multiwell strip comprising a frame portion consisting of a first stiff material, and a plurality of wells arranged in a line and consisting of a second material, which is not as stiff as the first material.

### SUMMARY

The invention provides a multi-well plate comprising a frame portion made of a first material, wherein the frame has a planar top surface having an array of a plurality of holes arranged in a columnar pattern (forming a columnar pattern of a plurality of holes) on the top surface, the frame further comprising an array of segmented breaking features arranged in a columnar pattern (forming a columnar pattern of segmented breaking features) alternating with the columnar pattern of the plurality of holes, and a plurality of wells placed into the plurality of holes on the top surface of the frame, wherein the wells are made of a second material and wherein the multi-well plate is manually divisible along a center section of the segmented breaking features into one or more columns of multi-well strips, wherein the segmented breaking feature comprises an array of petal shaped cuts that extend from the top surface of the frame through a bottom surface of the frame and a series of undercuts on a bottom surface of the frame portion, arranged such that the undercuts alternate with the petal shaped cuts.

In some embodiments, multi-well plates of the invention, are manually divisible along the center of the segmented breaking features into a plurality of single columns of multi-well strips. Multi-well strips can also be divided into segments comprising at least two columns of multi-well strips.

In some embodiments, the undercuts are V-shaped undercuts.

In some embodiments of a multi-well plate of the invention, petal shaped cuttings on the top surface of the frame combined with an undercut on the bottom surface of the frame creates eight small breaking sections or breaking features on each side of a column of multi-well plates. The length and thickness of each breaking section is the same across the frame. This pattern of breaking sections results in a segmented breaking pattern.

Advantageously, the segmented breaking pattern/feature, in contrast to continuous break lines that are present in some existing multi-well plates, provides one or more of the following improvements: eliminates the need of a tool for dividing the multi-well plate, and/or allows for manual tearing of the multi-well plate into strips/segments, and/or radius of petal shape and the localized breaking minimizes sharp edges after breaking and/or reduces sharp, jagged edges after breaking, and/or results in smooth edges and/or results in reducing the likelihood to cause glove tears or user hand and finger injuries.

The breakable feature design of the present inventionprevents end user's gloves from tearing and/or prevents injury such as cuts and nicks to fingers and hands of a user.

In some embodiments, multi-well plates of the invention, when manually divided into strips or segments of tubes or wells, provide or result in smooth edges, and/or minimize or reduce sharp edges and/or significantly reduce sharp or jagged edges. In some embodiments, manually dividing a multi-well plate of the inventionresults in one or more columns of multi-well strips that have no sharp edges. Accordingly, multi-well plates of the inventioncan be divided into strips or segments with reduced injury and/or no injury to a user. Multi-well plates of the inventioncan be divided into strips or segments with reduced or no glove tearing incidences.

In some embodiments, multi-well plates of the inventioncomprise a frame made of a first rigid material and the wells are made of a second softer material. In some embodiments, the first rigid material comprising the frame is polycarbonate, or an amorphous plastic and a partially crystalline heavily filled plastic and combinations thereof. In some embodiments, the second softer material comprising the wells is polypropylene, silicone, LSR (Liquid silicone rubber), or made of a soft plastic and partially crystalline plastic and combinations thereof. In some embodiments, the first rigid material comprising the frame is polycarbonate and the second softer material comprising the wells is polypropylene.

In some embodiments, wells of a multi-well plate of the invention, are directly molded into the frame utilizing form locking connections. In exemplary embodiments, molding and mechanical interlocking of parts on the underside of the frame forms the locking connections to join the wells to the frame.

In some embodiments, multi-well plates comprise 24, 48, 96, 384 or 1536 or more wells. In some embodiments, multi-well plates comprise 96 wells. In some embodiments, a multi-well plate of the invention can be manually divided into one or more columns of 8 tube strips or into segments comprising at least one column of 8-tube strips.

In some embodiments, multi-well plates of the invention, are compatible with one or more of the following including but not limited to: automation, manufacturing processes, lyophilization procedures, filling of reagents, freezing reagents, use in thermal cycling instruments, use in microbial processes and the like.

Multi-well plates of the inventionand the corresponding 8-tube strips and/or segments comprising at least two or more 8-tube strips are compatible with several commercially available thermal cycler instruments.

Multi-well plates of the invention, in some embodiments, can be sealed to enclose reagents. Seals can comprise but are not limited to one or more of peelable foil, clear seals, clear caps, optical seals, optical caps, domed caps, flat caps and the like. Frames, skirts and wells of the multi-well plates of the inventioncan additionally comprise features that fit and interface with a variety of automation devices and can have features to enable robotic gripping.

In some embodiments, a skirt feature is located extending from the edges along the frame of a multi-well plate either on parts of the frame or all along the frame. In some embodiments, multi-well plates of the invention, are of a standard profile design wherein the height of the skirt is of standard height, which allows tubes/wells of standard height to fit into the frame. In such embodiments, the skirt has an extended lip (sometimes referred to in the art as the SBS (Society for Biomolecular Screening) lip) that that wraps all around the perimeter of the skirt and plate. Such plates, in some embodiments, allow standard height multi-well tubes to be associated with the frame.

In some embodiments, multi-well plates of the invention, are low profile in design, wherein the height of the skirt is shorter than the standard height, which allows low profile tubes/wells to fit into the frame. In such embodiments, the skirt lip (referred to as SBS lip) is only provided at the four corners of the skirt of a multi-well plate.

Both standard profile and low profile multi-well plates of the inventionand their divisible strips and segments are useful in commercially available thermocycler instruments such as those used to perform polymerase chain reaction (PCR) and quantitative PCR (qPCR). Another advantage of the multi-well plates of the present inventionis therefore provision of a consumable multi-well plate with either pre-aliquoted, frozen or lyophilized reagents that are usable with common thermocycler instruments available in the market. One strip or segments with more than one strip can be used variously with different thermal cyclers.

These and other features of the present teachings will become more apparent from the detailed description in sections below.

### BRIEF DESCRIPTION OF THE DRAWINGS

The present disclosure may be better understood in reference to one or more the drawings below. The skilled artisan will understand that the drawings, described below, are for illustration purposes only. The drawings are not intended to limit the scope of the present teachings in any way.
Figure 1 shows a perspective view of a frame portion of a multi-well plate according to an example embodiment.
Figure 2 shows front view of the frame of Figure 1, with the dotted circled portion depicting an undercut on the bottom surface of the frame.
Figure 3 shows an exploded view of the dotted circled portion of Figure 2, enlarging the undercut on the bottom surface of the frame.
Figure 4 shows top perspective view of the frame of Figure 1, with the dotted circled portion depicting an edge on the frame.
Figure 5 shows an exploded view of the circled portion of Figure 4, describing an edge on the frame.
Figure 6 shows a bottom view of the frame of Figure 1, with the dotted circled portion depicting one breaking feature on the frame.
Figure 7 shows a perspective view of a multi-well plate, showing a frame portion as shown in Figure 1 and tubes or wells inserted therein, according to an example embodiment.
Figure 8 shows front view of the multi-well plate of Figure 7, with the dotted circled portion depicting an undercut on the bottom surface of the frame.
Figure 9 shows an exploded view of the dotted circled portion of Figure 8, describing the undercut on the bottom surface of the frame.
Figure 10A shows a cross section view of a tube/well interlocking into a frame and depicts a portion of the frame interlocking with a portion on the tube/well.
Figure 10B shows a perspective view of a tube/well, depicted without a frame, and shows frame interlocking portions including a chimney and a lug.
Figure 10C shows a cross-section view of a tube/well, depicted without a frame, and shows frame interlocking portions including a chimney and a lug.
Figure 11 shows top perspective view of the multi-well plate of Figure 7, with the circled portion depicting an edge on the frame portion thereof.
Figure 12 shows an exploded view of the dotted circled portion of Figure 11, describing an edge on the frame portion thereof.
Figure 13 shows bottom view of the multi-well plate of Figure 7, depicting an array of segmented breaking features in columnar arrangement thereon.
Figure 14 shows a perspective view of a multi-well plate as shown in Figure 7 with one tube-strip or well-strip being torn away therefrom, according to an example embodiment.
Figure 15 depicts a perspective view if one tube strip or well-strip that has been torn or divided out of a multi-well plate as shown in Figures 7 and 14, according to an example embodiment.
Figures 16A and 16B depicts a right side and a left side view respectively of the tube strip or well-strip of Figure 15, according to an example embodiment.
Figures 17A and 17B depicts a top and a bottom view respectively of the tube strip or well-strip of Figure 15, according to an example embodiment.
Figure 18 shows a perspective view of a frame portion of a multi-well plate, according to another example embodiment.
Figure 19 shows front view of the frame of Figure 18, with the dotted circled portion depicting an undercut on the bottom surface of the frame.
Figure 20 shows an exploded view of the dotted circled portion of Figure 19, describing the undercut on the bottom surface of the frame.
Figure 21 shows top perspective view of the frame of Figure 18, with the circled portion depicting an edge on the frame.
Figure 22 shows an exploded view of the dotted circled portion of Figure 21, describing an edge on the frame.
Figure 23 shows bottom view of the frame of Figure 18, with the circled portion depicting one breaking feature on the frame.
Figure 24 shows a perspective view of a multi-well plate showing a frame portion as shown in Figure 18 and tubes or wells inserted therein, according to an example embodiment.
Figure 25 shows front view of the multi-well plate of Figure 24, with the circled portion depicting an undercut on the bottom surface of the frame.
Figure 26 shows an exploded view of the dotted circled portion of Figure 25, describing an undercut on the bottom surface of the frame.
Figure 27A shows a cross section view of a tube/well interlocking into a frame and depicts a portion of the frame interlocking with a portion on the tube/well.
Figure 27B shows a perspective view of a tube/well, depicted without a frame, and shows frame interlocking portions including a chimney and a lug.
Figure 27C shows a cross-section view of a tube/well, depicted without a frame, and shows frame interlocking portions including a chimney and a lug.
Figure 28 shows top perspective view of the multi-well plate of Figure 24, with the dotted circled portion depicting an edge on the frame portion thereof.
Figure 29 shows an exploded view of the dotted circled portion of Figure 28, depicting an edge on the frame portion thereof.
Figure 30 shows bottom view of the multi-well plate of Figure 24, depicting an array of segmented breaking features in columnar arrangement thereon.
Figure 31 shows a perspective view of a multi-well plate as shown in Figure 24 with one tube-strip or well-strip being torn away therefrom, according to an example embodiment.
Figure 32 depicts a perspective view if one tube strip or well-strip that has been torn or divided out of a multi-well plate as shown in Figure 24 or Figure 31, according to an example embodiment.
Figures 33A and 33B depicts a right side and a left side view respectively of the tube strip or well-strip of Figure 32, according to an example embodiment.
Figures 34A and 34B depicts a top and a bottom view respectively of the tube strip or well-strip of Figure 32, according to an example embodiment.

### DETAILED DESCRIPTION

It is to be understood that both the foregoing general description and the following detailed description are exemplary and explanatory only and are not intended to limit the scope of the current teachings. In this application, the use of the singular includes the plural unless specifically stated otherwise. Also, the use of "comprise", "contain", and "include", or modifications of those root words, for example but not limited to, "comprises", "contained", and "including", are not intended to be limiting. Use of "or" means "and/or" unless stated otherwise. The term "and/or" means that the terms before and after can be taken together or separately. For illustration purposes, but not as a limitation, "X and/or Y" can mean "X" or "Y" or "X and Y".

The multi-well plate of the invention is manually divisible into strips of wells or tubes that may be compatible with reagent dispensing by human or automation systems in manufacturing environments. In some examples, the present multi-well plates are useful to produce kits for various biological or chemical processes, wherein the kit reagents comprised in a multi-well plate of the present invention, can be produced in a high-throughput environment and sold to low throughout consumers/users. Accordingly, multi-well plates of the invention advantageously allow prevention of wastage of expensive reagents.

In some embodiments, multi-well plates of the invention, are compatible with one or more of the following including but not limited to: automation, manufacturing processes, lyophilization procedures, filling of reagents, freezing reagents, use in thermal cycling instruments, use in microbial processes and the like.

Multi-well plates of the invention can be used for reagent storage and/or downstream processing of reactions such as but not limited to nucleic acid amplification, nucleic acid sequencing, sample preparation to extract biomolecules of interest including nucleic acids, proteins, carbohydrates, lipids among others, growing and culturing microbes.

Some example embodiments described herein provide divisible multi-well plates that can be divisible in to 8-tube strips or segments of one, two, three or more 8-tube strips, as required by an end user. Multi-well plates of the invention and the corresponding 8-tube strips and/or segments comprising at least two or more 8-tube strips are compatible and therefore mechanically fit and interface with several commercially available thermal cycler instruments, nucleic acid sequencing devices, sample preparation devices, microbial incubators and other bioprocess devices.

As defined in the claims, the present inventionprovides a multi-well plate comprising: a frame portion made of a first material, wherein the frame portion has a planar top surface having an array of a plurality of holes arranged in a columnar pattern on the top surface, the frame portion further comprising an array of segmented breaking features arranged in a columnar pattern alternating with the columnar pattern of the plurality of holes and a plurality of wells made of a second material, wherein the wells are placed into the plurality of holes on the top surface of the frame, wherein the multi-well plate is manually divisible along the center of the segmented breaking features into one or more columns of multi-well strips.

Multi-well plates of the disclosure, are manually divisible along the center of the segmented breaking features into a plurality of single columns of multi-well strips. Multi-well strips can also be divided into segments comprising at least two columns of multi-well strips.

The segmented breaking features of the multi-well plates of the invention, comprise a plurality of petal shaped cuts that extend from the top surface of the frame portion through a bottom surface of the frame and a plurality of undercuts on a bottom surface of the frame portion, arranged such that the undercuts alternate with the petal shaped cuts. In some embodiments, the undercuts are V-shaped undercuts.

In some embodiments of a multi-well plate of the invention, petal shaped cuttings on the top deck of the frame combined with an undercut on the bottom of the frame creates eight small breaking sections or breaking features on each side of a column of multi-well plate. The length and thickness of each breaking section is the same across the frame. This pattern of breaking sections results in a segmented breaking pattern.

Advantageously, the segmented breaking pattern/feature, in contrast to continuous break lines that are present in some existing multi-well plates, provides one or more of the following improvements: eliminates the need of a tool for dividing the multi-well plate, and/or allows for manual tearing of the multi-well plate into strips/segments, and/or minimizes sharp edges after breaking due to the radius of petal shape cuts and the localized breaking and/or reduces sharp, jagged edges after breaking, and/or results in smooth edges and/or results in reduced incidences of glove tearing and injury to users.

In some embodiments, a skirt feature is located extending from the edges along the frame of a multi-well plate either on parts of the frame or all along the frame. In some embodiments, the skirt further has an extended lip that wraps all around the perimeter of the skirt and frame of plate. The lip is also described in the art as the SBS (Society for Biomolecular Screening) lip or as the SLAS (Society for laboratory Automation and Screening) lip, which is according to the ANSI (American National Standards Institute) for micro-well plates.

Multi-well plates of the present invention, in some embodiments, are low profile plates, wherein the skirt is shorter to accommodate tubes/wells of lower volumes. In some embodiments, low-profile tubes or well have a maximum volume of 0.2mL when fully filled. Typical height of low profile tubes is about 15.50 ± 0.25 mm. In some embodiments, low profile multi-well plates of the inventionhave a skirt lip, which is an extension of the skirt and is located on the four corners of the frame of a multi-well plate.

In other embodiments, multi-well plates of the invention, are standard profile plates, wherein the height of the skirt is of standard height, which allows tubes/wells of standard height to fit into the frame. In such embodiments, the skirt has an extended lip (the SBS or SLAS lip) that that wraps all around the perimeter of the skirt and plate. Such plates, in some embodiments, allow standard height multi-well tubes to be associated with the frame. Standard height or standard profile wells or tubes have a maximum well volume of 0.3mL when used with adhesive and heat seals. Typical height of standard profile tubes or wells is about 20.70 ±0.25 mm.

Both standard profile and low profile multi-well plates of the inventionand their divisible strips and segments are useful in commercially available thermocycler instruments such as those used to perform polymerase chain reaction (PCR) and quantitative PCR (qPCR). In non-limiting examples, the present semi-skirted multi-well plates and strips and segments derived therefrom mechanically fit and interface with exemplary thermocycler instruments such as Applied Biosystems 7500, QuantStudio 3 QuantStudio 5, QuantStudio 6, QuantStudio 7, Applied Biosystems ProFlex, Veriti, SimpliAmp, among others. The multi-well plates of the invention also fit and interface with heat sealers and aluminum support racks.

Multi-well plates of the present invention, in some embodiments, can fit and interface with other instruments such as sequencing instruments and platforms, sample preparation instruments and robotic platforms and the like.

Another advantage of the multi-well plates of the present invention is therefore provision of a consumable multi-well plate with or without one or more of a pre-aliquoted, a frozen and/or a lyophilized reagent that are usable with common thermocycler instruments and other commonly used instruments and devices available in the market. A single strip or a segment comprising more than one strip can be used variously with different thermal cyclers.

The embodiments described below are exemplary embodiments with reference to the drawings. Like reference numerals and characters in the drawings refer to like elements or equivalents. However, the disclosure is not to be construed in limitation to the drawings and specific embodiments which are mere examples of concepts. For example, while most of the drawings depict 96-well plates that have 8×12=96 tubes/wells arranged in rows and columns, one of skill in the art, in light of the teachings of the present disclosure, will realize that these teachings extend to multi-well plates having more or fewer wells/tubes such as but not limited to multi-well plates with 24, 48, 96, 384 or 1536 or more wells/tubes.

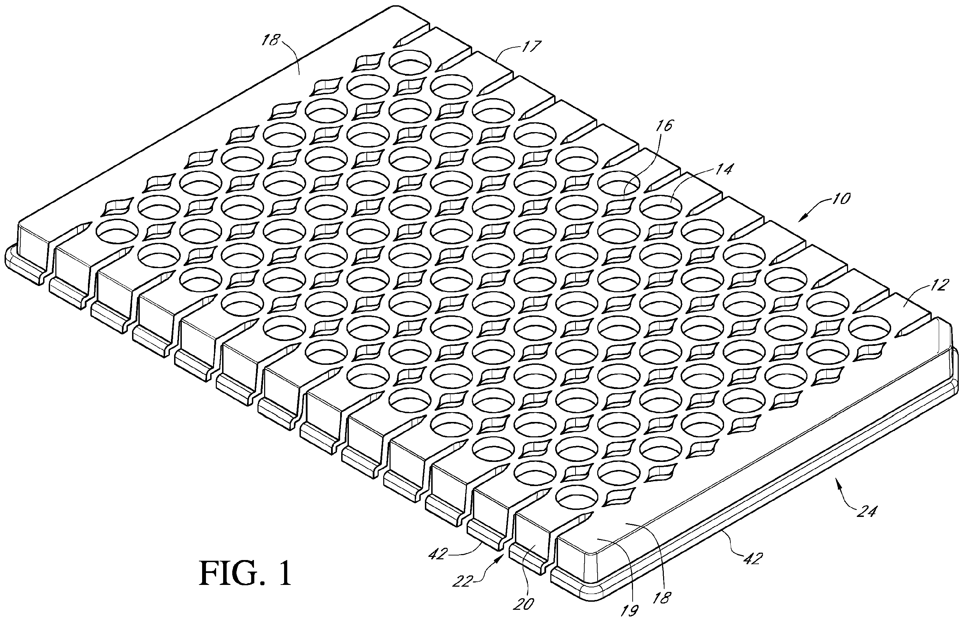

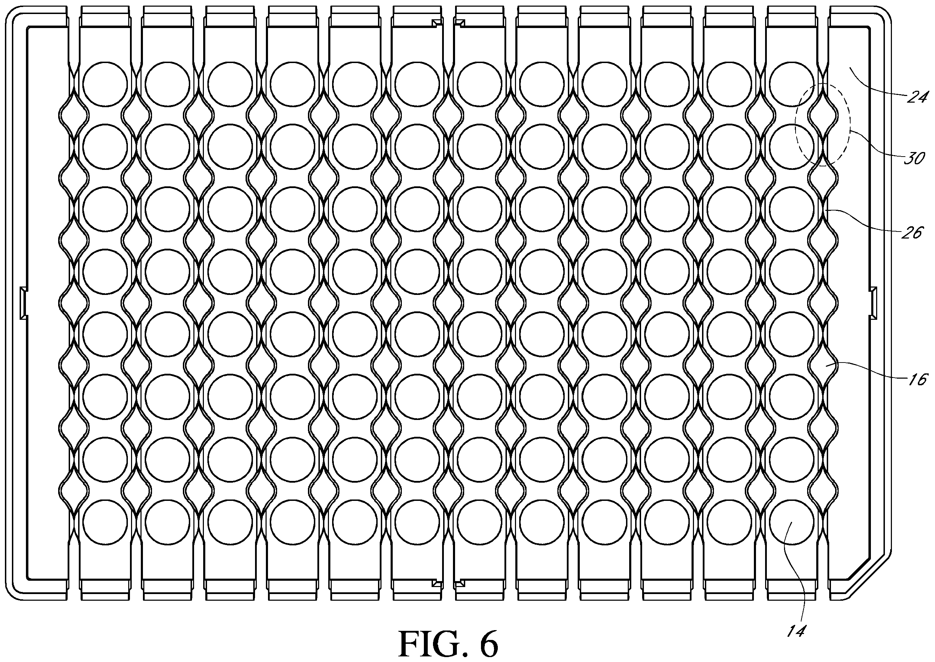

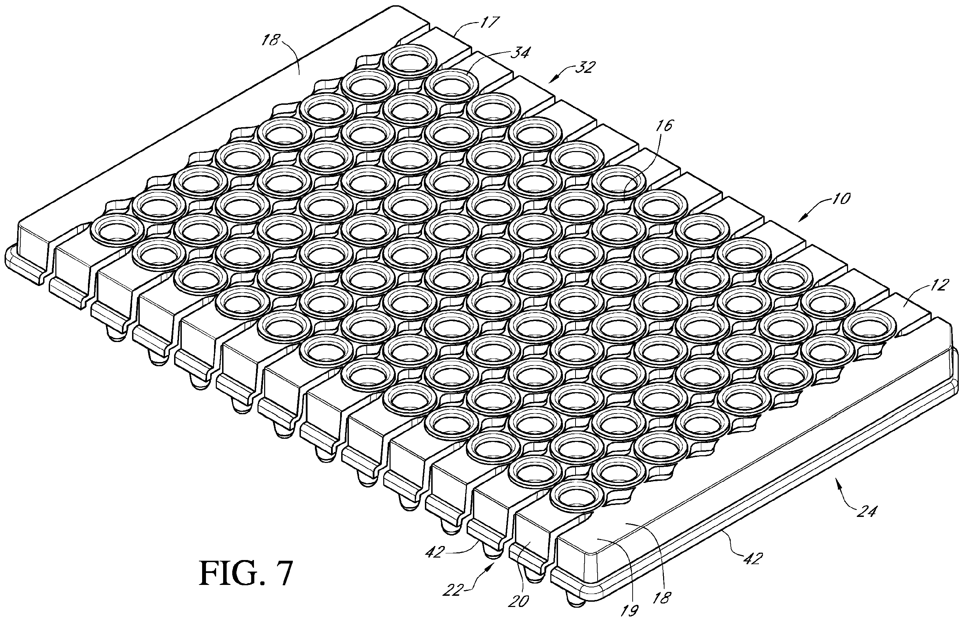

Figure 1 shows a perspective view of a frame portion 10 of a multi-well plate according to an example embodiment. Frame 10 comprises a planar top surface 12 having an array of a plurality of holes 14 arranged in a columnar pattern on the top surface 12. Holes 14 are configured to receive therein tubes or wells 34 (tube/well 34 are depicted later, for example in Figure 7). Frame portion 10 further comprises an array of petal shaped features 16 arranged in a columnar pattern alternating with the columnar pattern of the plurality of holes 14. Petal shaped features 16 are part of breaking features 30 described in further detail in sections below, for example see Figure 6.

Figure 1 also shows two edges 18 located on the left and right sides of the array of holes 14 and petal shaped features 16. Edges 18 can have alpha numeric labeling to mark or label holes 14, which are configured to receive therein tubes or wells 34 (tubes 34 are depicted in Figure 7). In one non-limiting example, right edge 18 can have alphabetical numbering such as A, B, C, D, E, F, G, H, etc. Numeric labeling is also alternatively possible. Labeling may also be done on left edge 18.

Figure 1 also shows top side 17 and bottom side 19 of the top planar surface 12 with a plurality of skirts 20 extending therefrom. A plurality of cuts 22 separate each column comprising holes 14 at the top side 17 and bottom side 19. Cuts 22 are aligned with petal shaped features 16 in a straight line. Columns comprising plurality of holes 14 are additionally defined on the two ends of the column by two cuts 22 can also be alphanumerically labelled on the top side 17 of Figure 1.

In non-limiting examples, columns formed in between the plurality of cuts 22 can be labeled or marked on the top side 17 of frame 10 by numbers such as 1, 2, 3, 4, 5, 6, 7, 8, 9, 10, 11, 12, *etc.* These columns comprise a plurality of holes 14 arranged linearly. Alphabetical labeling is also alternatively possible for these columns. Labeling may also be dome on bottom side 19. Such labeling helps identify the tubes/wells and the reactions therein. For example, in the non-limiting labeling example above, a first tube 34 inserted on the top right side of frame 1 can be identified by the label A1.

Figure 1 also depicts skirt lip 42, which according to this embodiment, wraps around the perimeter of the entire frame (with the exception of cuts 22). As can be seen skirt lip 42 extends from skirt 20. In this embodiment, skirt 20 is longer in height (as compared to another embodiment described later) and accommodate standard size tubes/wells. Hence frames with standard height skirt portions are described as standard profile multi-well plate frame. The embodiment of Figure 1, with a skirt lip 42 wrapping all-around the perimeter of the plate frame (with the exception if cuts 22) is referred to as a skirted frame.

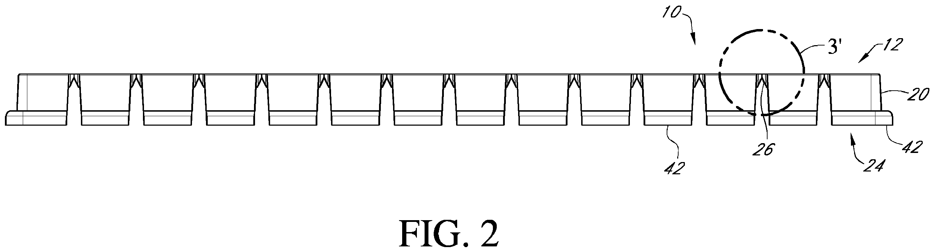

Figure 2 shows a front view of frame portion 10 of a multi-well plate of the invention, wherein the circled portion depicts an undercut 26 located on the bottom surface 24 of frame 10. Undercut 26 and petal shaped features 16 are both comprised in breaking features 30 (shown in Figure 6). Breaking features 30 are arranged as segmented breaking features.

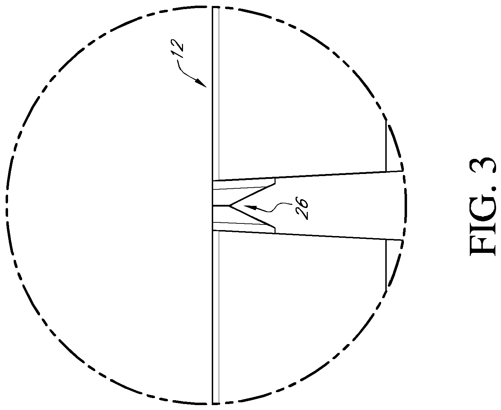

Figure 3 shows an exploded view of the dotted circled portion of Figure 2, describing undercut 26 located on bottom surface 24 of frame 10. In some embodiments, undercut 26 is V-shaped.

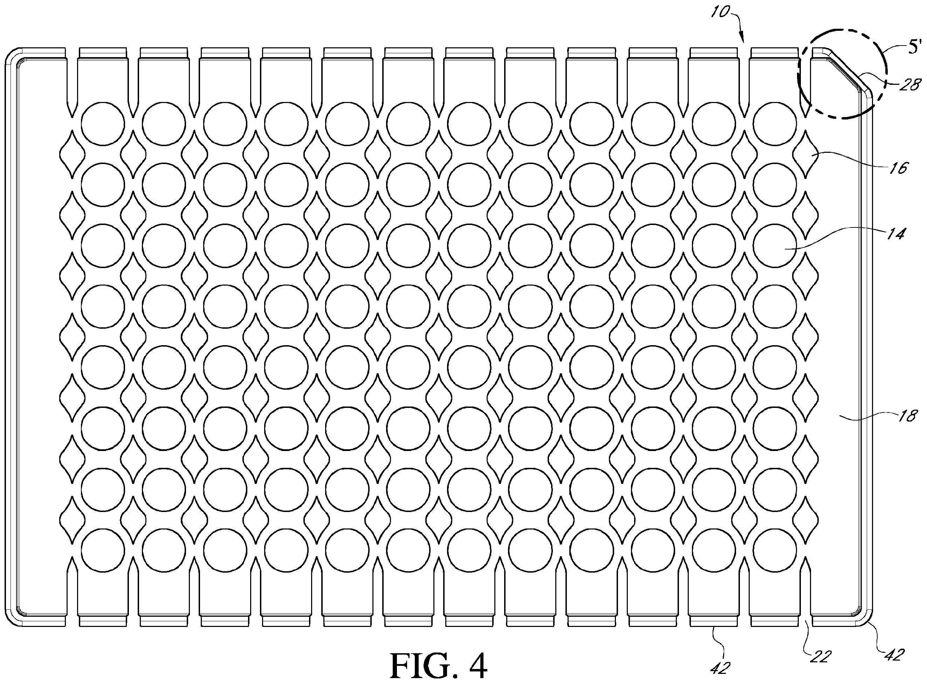

Figure 4 shows a top perspective view of frame portion 10 of a multi-well plate, with the dotted circled portion depicting an edge 28 of frame 10.

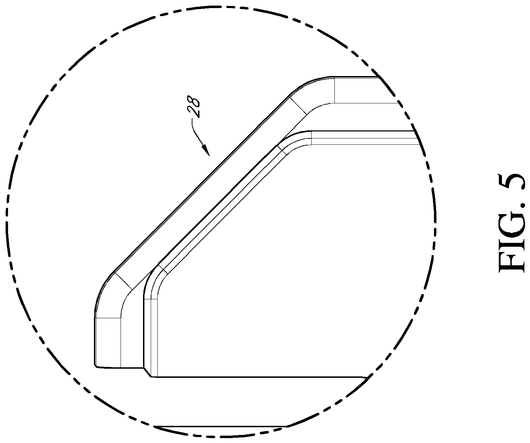

Figure 5 shows an exploded view of the dotted circled portion of Figure 4, describing the depicting an edge 28 on frame 10. Edge 28 on frame 10 can be used for plate orientation in instruments where the multi-well plates are used including automated filling instruments, PCR instruments and the like.

Figure 6 shows bottom view of frame portion 10 of Figure 1, with the dotted circled portion depicting one breaking feature 30 formed on the frame. As noted above, one breaking feature 30 comprises a petal shaped feature 16 and an undercut 26. A plurality of breaking features 30 are formed beginning from one cut 22 located on one end (e.g. tops side 17) of frame 10 and going along a linear path to another cut 22 located the opposite side of frame 10 (e.g., bottom side 19). This pattern forms a plurality of segmented breaking features 30. Segmented breaking features 30 allow for manual tearing with smooth edges, with no rough edges or with significantly reduced rough or jagged ends.

Figure 7 shows a perspective view of top surface 12 of a multi-well plate 32 showing a frame portion 10 (of standard profile as shown in Figure 1) and tubes or wells 34 inserted into holes 14 thereon, according to an example embodiment.

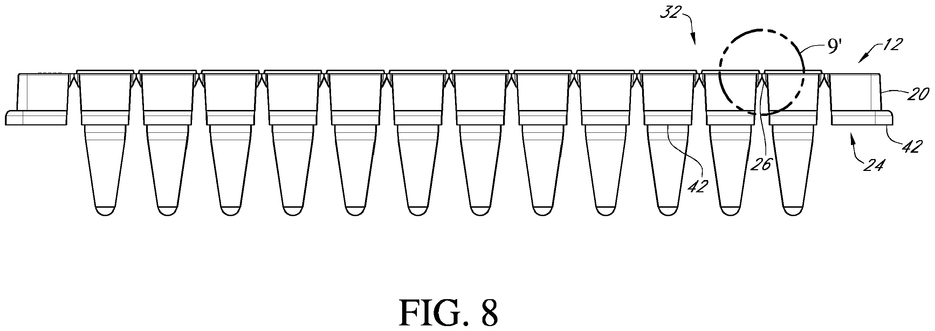

Figure 8 shows front view of the multi-well plate of Figure 7, with the dotted circled portion depicting an undercut 26 on the bottom surface 24 of the frame.

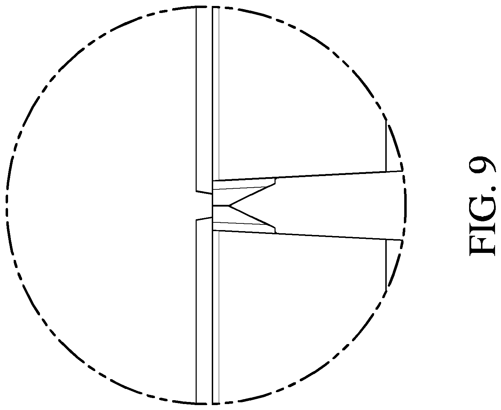

Figure 9 shows an exploded view of the dotted circled portion of Figure 8, describing undercut 26 on the bottom surface 24 of frame 10.

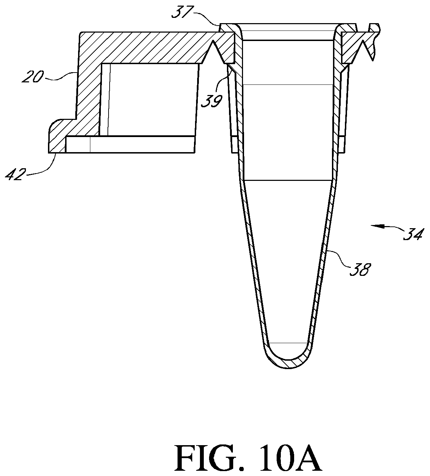

Figure 10A shows a cross section view of a well or tube 34 interlocking into a frame 10 and depicts a chimney 37 of the frame 10 interlocking with a lug 39 located on the side of tube/well 34. Skirt 20 and lip 42 are also shown in this figure.

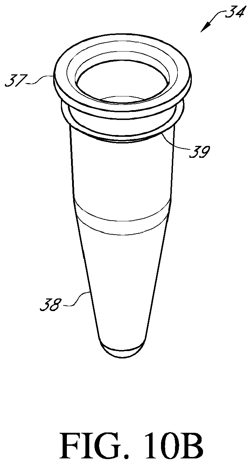

Figure 10B shows a perspective view of tube 34, depicted without frame 10, and shows chimney 37 and lug 39.

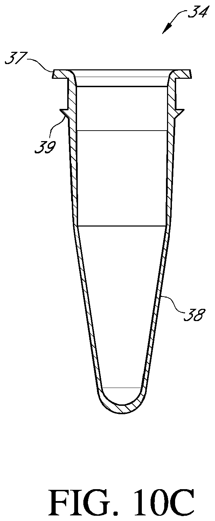

Figure 10C shows a cross-section view of tube/well 34, depicted without frame 10, and shows frame interlocking portions including a chimney 37 and a lug 39.

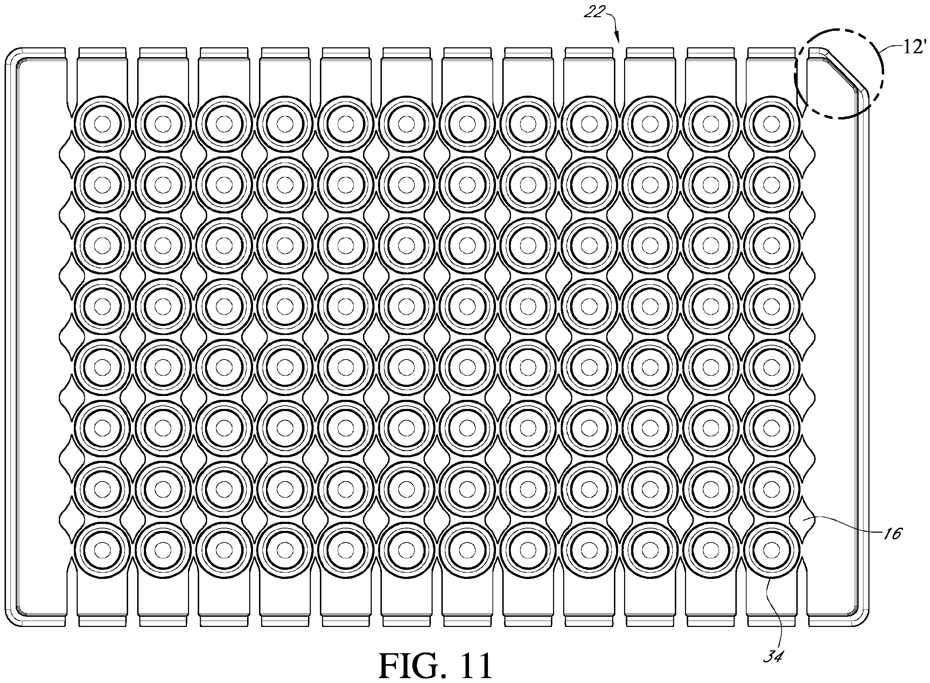

Figure 11 shows top perspective view of the multi-well plate of Figure 7, with the dotted circled portion depicting an edge 28 on frame portion 10 thereof.

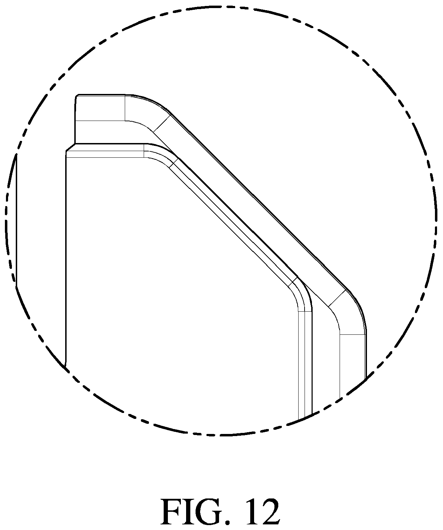

Figure 12 shows an exploded view of the dotted circled portion of Figure 11, describing the depicting edge 28 on frame portion 10 thereof.

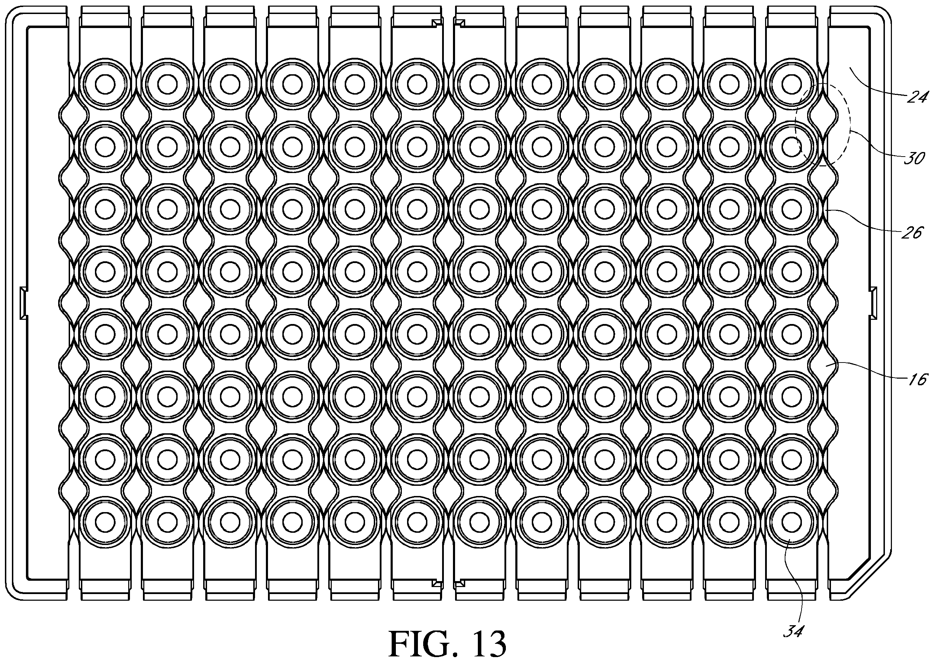

Figure 13 shows bottom view of multi-well plate 32 of Figure 7, having tubes 34 inserted in holes 14, depicting an array of segmented breaking features 30 in linear columnar arrangement on the bottom surface 24, extending between two cuts 22, wherein each cut 22 is located on opposite sides of each other on frame 10.

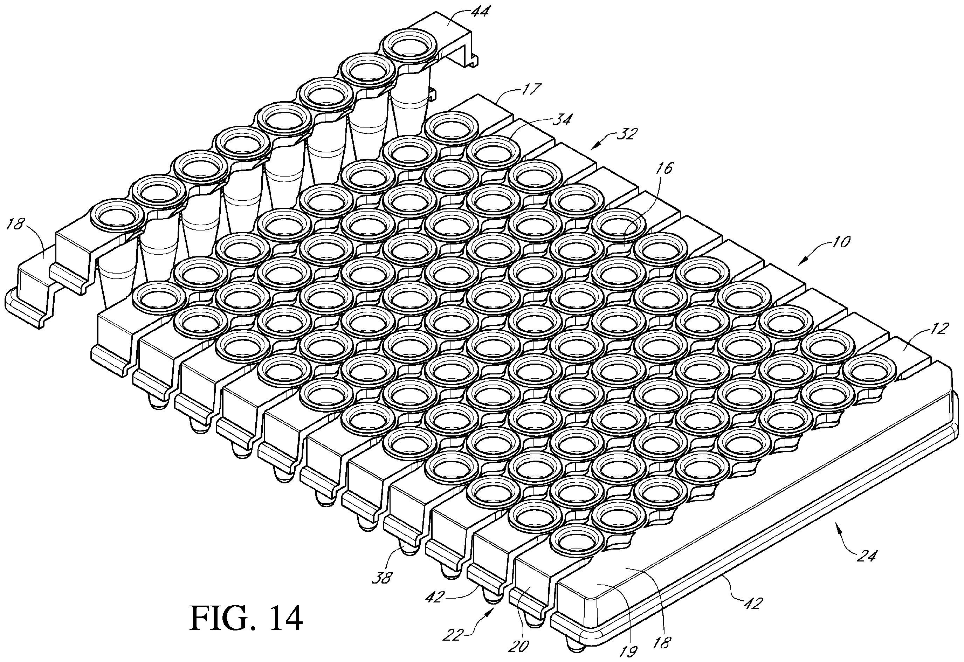

Figure 14 shows a perspective view of a multi-well plate 32 as shown in Figure 7 with one tube-strip or well-strip 44 being torn away therefrom, according to an example embodiment.

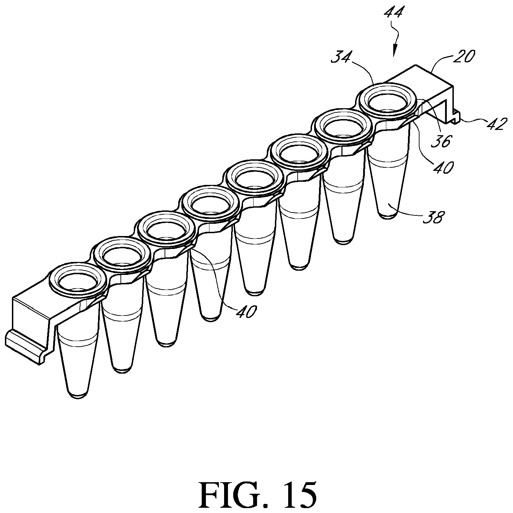

Figure 15 depicts a perspective view of one tube strip 44 (also referred to herein alternatively as well-strip 44) that has been torn or divided out of a multi-well plate 32 as shown in Figures 7 and 14, according to an example embodiment. Tube-strip 44 depicts an exemplary top portion or rim 36 of a tube 34. 38 depicts bottom portion of tube 34 and a plurality if torn edges 40. In some embodiments, torn edge 40 is a smooth edge. In some embodiments, torn edge 40 is not jagged. In some embodiments, torn edge 40 is not rough. In some embodiments, torn edge 40 has significantly reduced sharp edges or significantly reduced jagged edges as compared to other divisible or tearable plates that are commercially available. Skirt 20 and skirt lip 42 are seen on the side edges of tube strip 44.

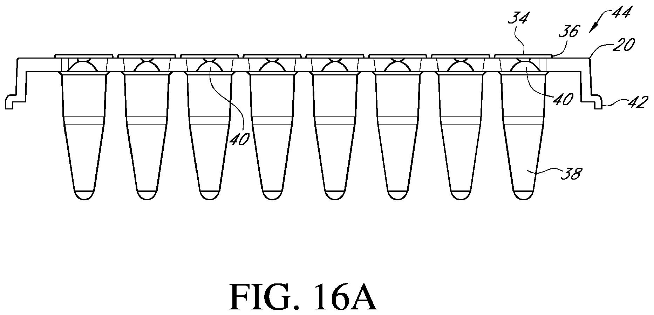

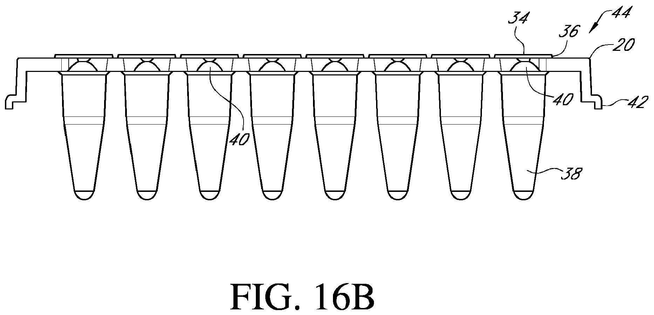

Figures 16A and 16B depict a right side and a left side view respectively of the tube strip 44 of Figure 15, according to an example embodiment and show among other features torn edges 40, skirt lip 42 and skirt 20.

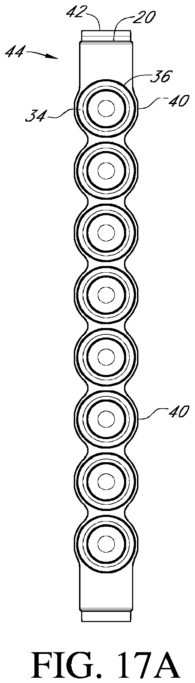

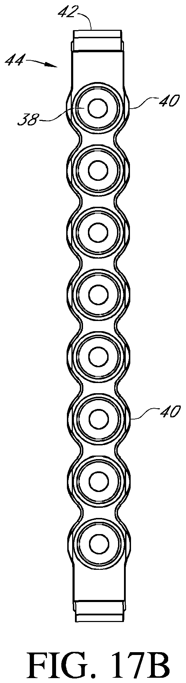

Figures 17A and 17B depict a top and a bottom view respectively of the tube strip or well-strip of Figure 15, according to an example embodiment.

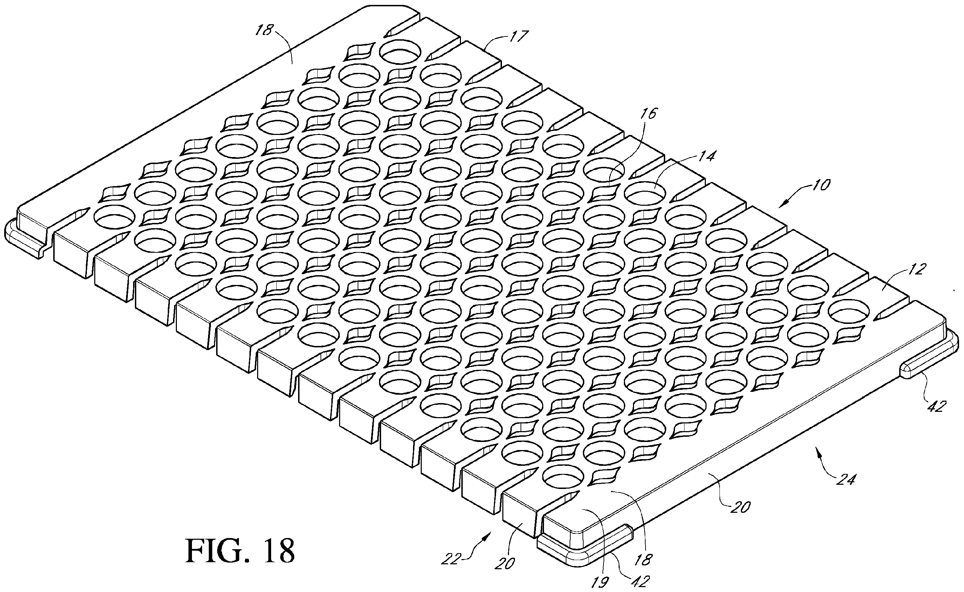

Figure 18 shows a perspective view of a frame portion 10 of a multi-well plate 32, according to another example embodiment. Figure 18 is very similar to Figure 1, except that the skirt portions 20 are shorter and skirt lip portion 42 is located only on the four corners of plate 32. Embodiments where skirt 20 is shorter and lip 42 is only located at the corners are described as low profile plates since they have shorter skirts 20 as compared to standard profile plate skirts 20 shown for example in Figures 1 and 7. Low profile plates accommodate lower volume tubes as compared to standard profile plates.

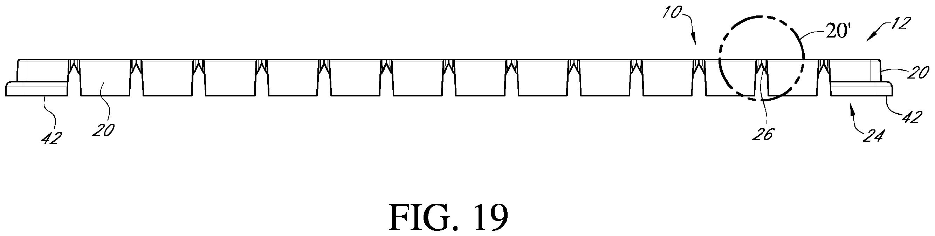

Figure 19 shows front view of frame portion 10 of the low profile multi-well plate 32 of Figure 18, with the circled portion depicting an undercut 26 on the bottom surface 24 of frame 10.

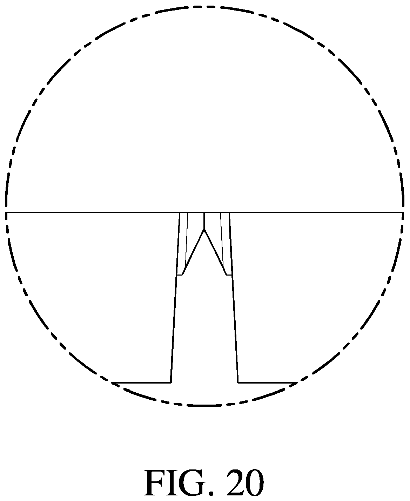

Figure 20 shows an exploded view of the dotted circled portion of Figure 18, describing undercut 26 on bottom surface 24 of frame 10.

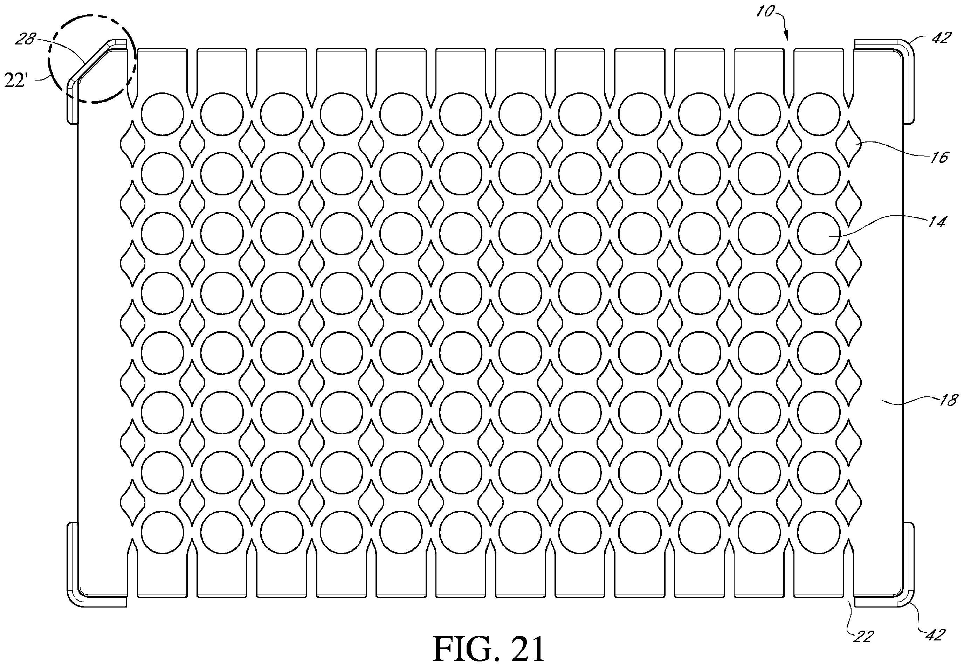

Figure 21 shows top perspective view of frame portion 10 of multi-well plate 32 of Figure 18, with the dotted circled portion depicting an edge 28 on the frame. Edge 28 is helpful for plate orientation in instruments.

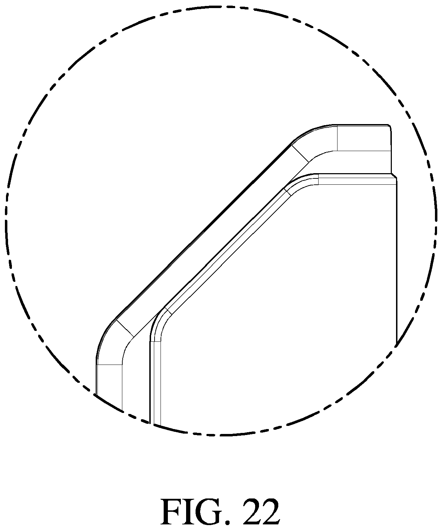

Figure 22 shows an exploded view of the dotted circled portion of Figure 21, describing the depicting edge 28 on frame 10.

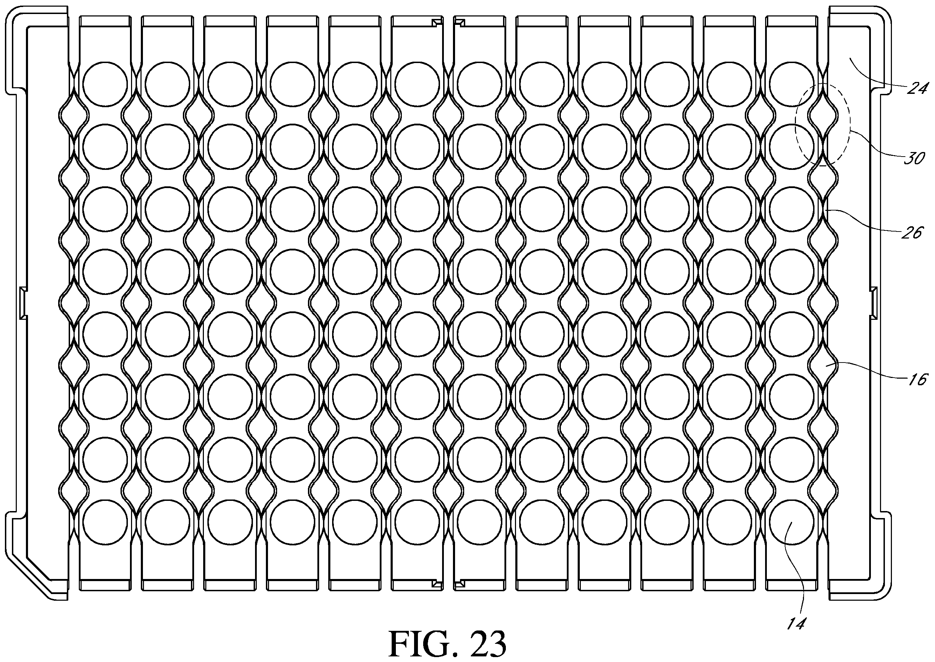

Figure 23 shows bottom view of frame portion 10 of the multi-well plate 32 of Figure 18, with the dotted circled portion depicting one breaking feature 30 on frame 10.

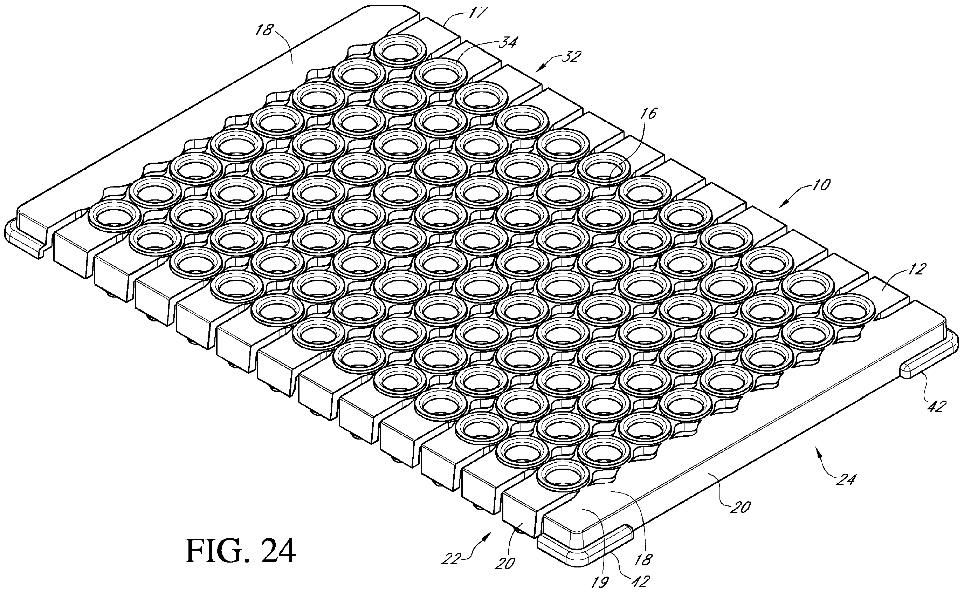

Figure 24 shows a perspective view of a multi-well plate showing a low profile skirted 20 frame portion 10 as shown in Figure 18 and tubes or wells 34 inserted in holes 14 located on top surface 12 of the plate, according to one example embodiment.

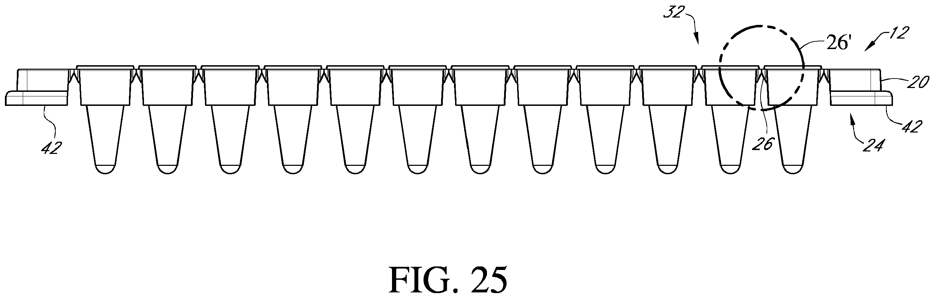

Figure 25 shows front view of the multi-well plate 32 of Figure 24, with the dotted circled portion depicting undercut 26 on bottom surface 24 of frame 10.

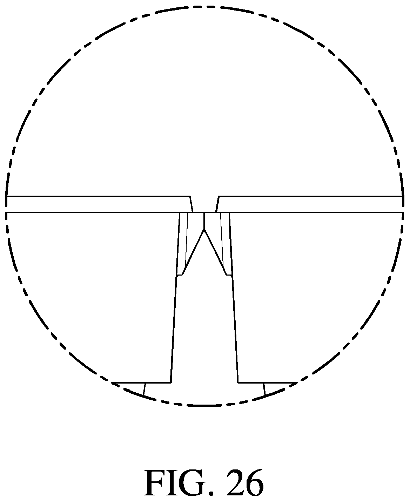

Figure 26 shows an exploded view of the dotted circled portion of Figure 25, describing undercut 26 on bottom surface 24 of frame 10.

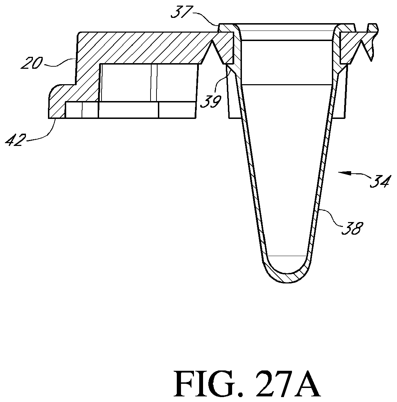

Figure 27A shows a cross section view of a low-profile multi-well plate showing a tube/well 34 interlocking into frame 10 and further depicts a portion of the frame, referred to as chimney 37 interlocking with a portion on the tube/well referred to as lug 39.

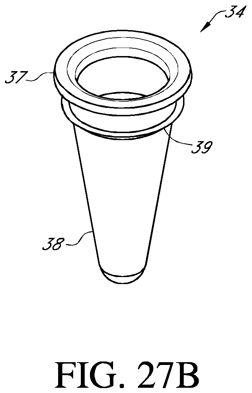

Figure 27B shows a perspective view of tube 34, depicted without frame 10, and shows chimney 37 and lug 39.

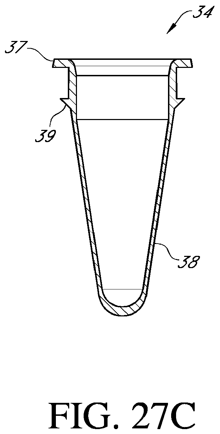

Figure 27C shows a cross-section view of tube/well 34, depicted without frame 10, and shows frame interlocking portions including a chimney 37 and a lug 39.

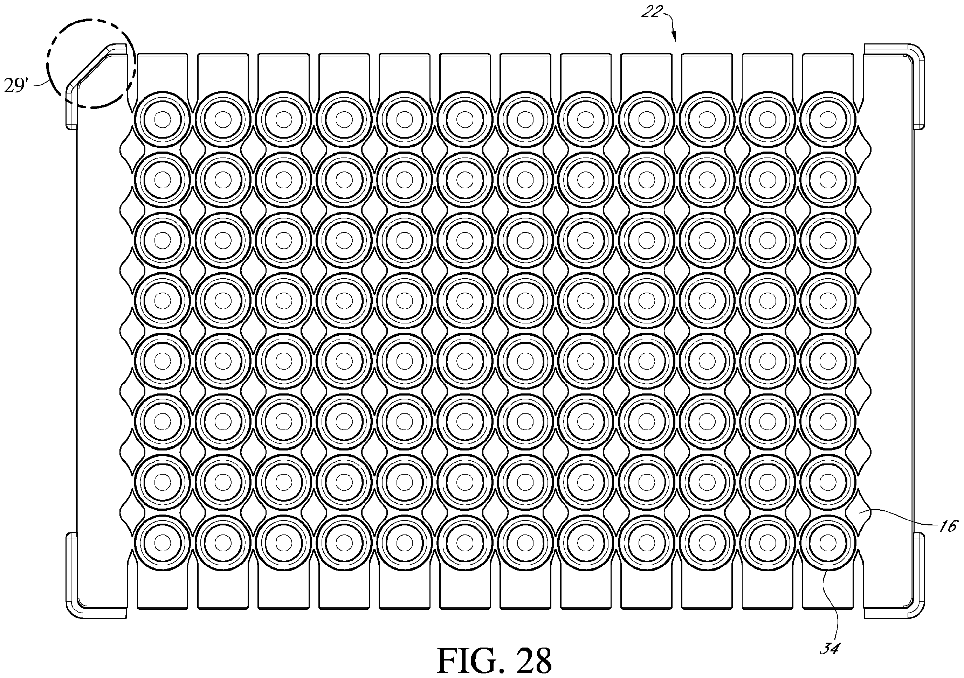

Figure 28 shows top perspective view of the multi-well plate 32 of Figure 24, with the dotted circled portion depicting an edge 28 on frame portion 10 thereof.

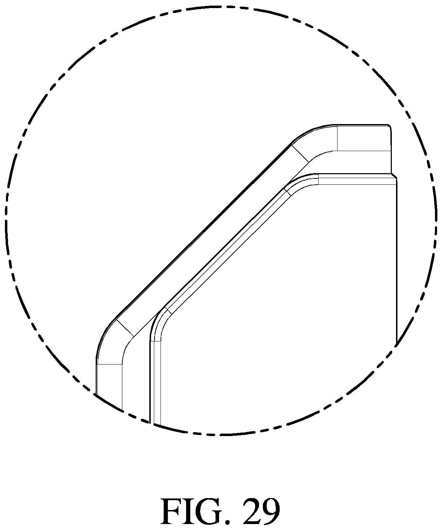

Figure 29 shows an exploded view of the dotted circled portion of Figure 28, describing edge 28 on frame portion 10 thereof.

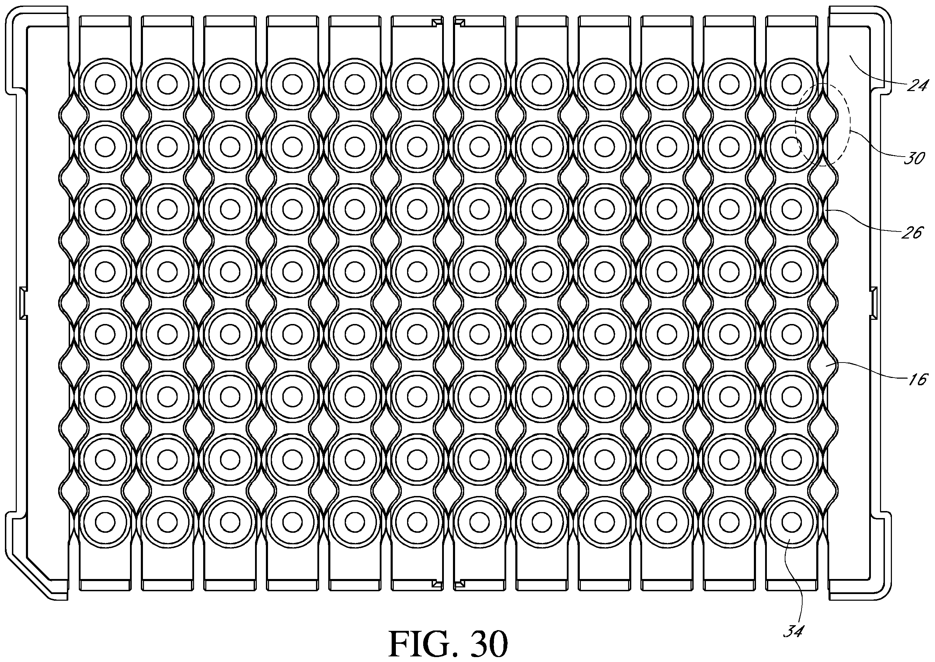

Figure 30 shows bottom view of the multi-well plate 32 of Figure 24, depicting an array of segmented breaking features 30 in a linear columnar arrangement thereon.

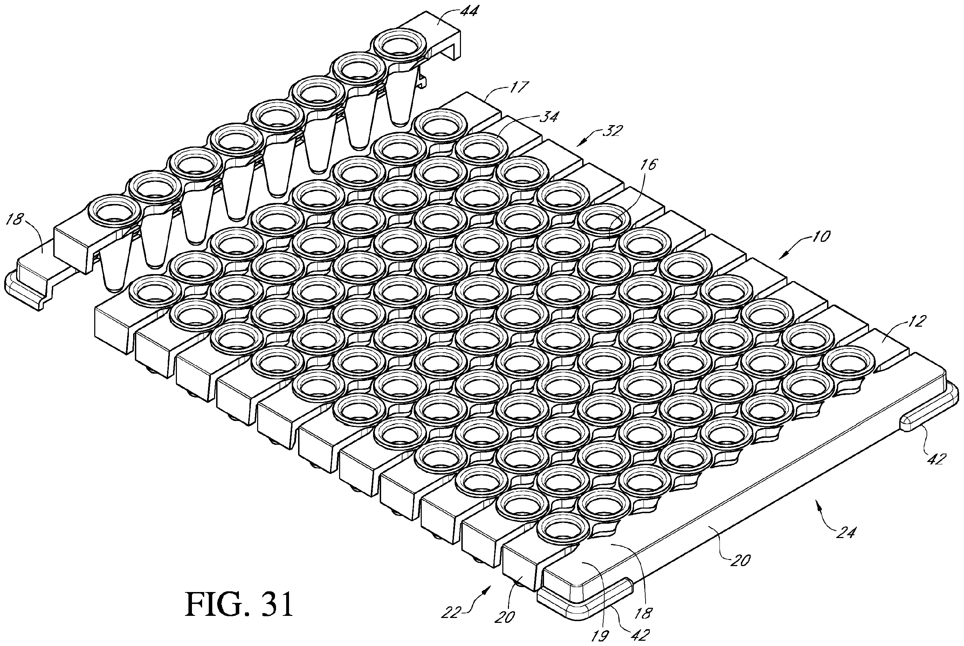

Figure 31 shows a perspective view of a multi-well plate 32 as shown in Figure 24 with one tube-strip 44 being torn away therefrom, according to an example embodiment.

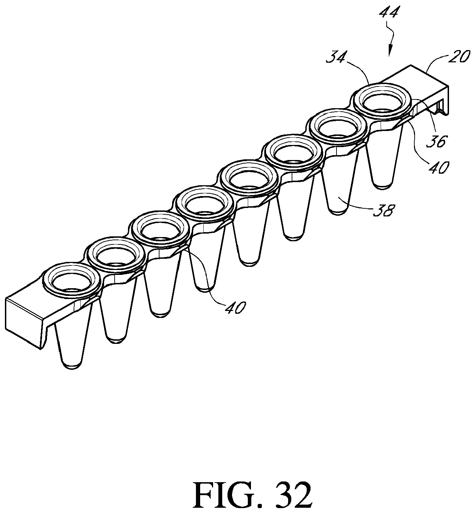

Figure 32 depicts a perspective view of one tube strip 44 that has been torn or divided out of a multi-well plate 32 as shown in Figures 24 and 31, according to an example embodiment. This tube strip 44 is a low-profile tube strip, having a shorter skirt 20 and lacking a skirt lip portion since it is derived from a low-profile multi-well plate that only has the skirt lip 42 located on the four corners of the plate frame.

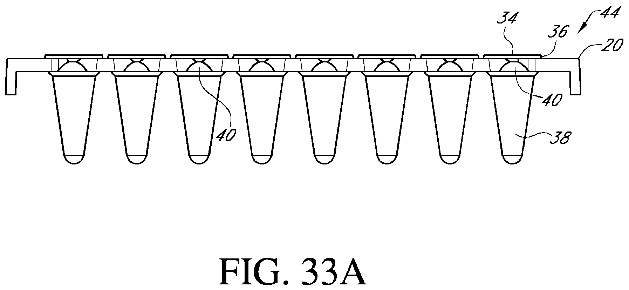

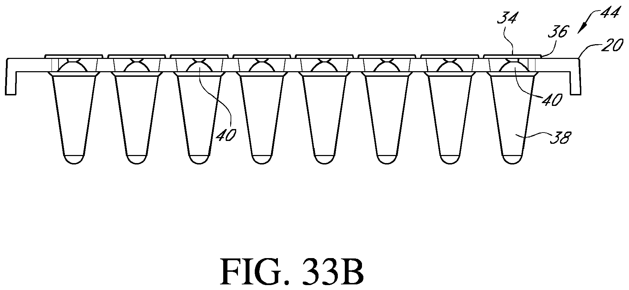

Figures 33A and 33B depicts a right side and a left side view respectively of the tube strip or well-strip 44 of Figure 32, according to an example embodiment, and depicts a torn edge 40. In some embodiments, torn edge 40 is a smooth edge. In some embodiments, torn edge 40 is not jagged. In some embodiments, torn edge 40 is not rough. In some embodiments, torn edge 40 has significantly reduced sharp edges or significantly reduced jagged edges as compared to other divisible or tearable plates that are commercially available.

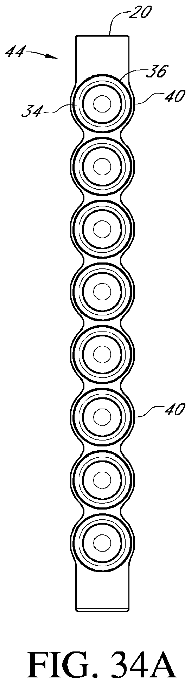

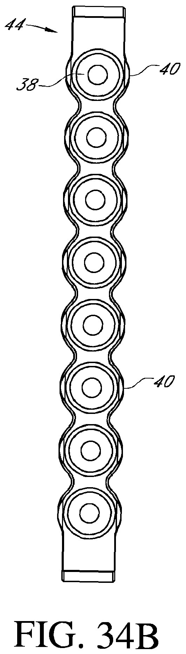

Figures 34A and 34B depicts a top and a bottom view respectively of the tube strip or well-strip 44 of Figure 32, according to an example embodiment.

## Claims

1. A multi-well plate comprising:
a frame made of a first material, wherein the frame has a planar top surface having an array of a plurality of holes arranged in a columnar pattern on the top surface, the frame further comprising an array of segmented breaking features arranged in a columnar pattern alternating with the columnar pattern of the plurality of holes; and
a plurality of wells made of a second material, wherein the wells are placed into the plurality of holes on the top surface of the frame,
wherein the multi-well plate is manually divisible along a center of the segmented breaking features into one or more columns of multi-well strips,
wherein the segmented breaking feature comprises an array of petal shaped cuts that extend from the top surface of the frame through a bottom surface of the frame and a series of undercuts on a bottom surface of the frame portion, arranged such that the undercuts alternate with the petal shaped cuts.

2. The multi-well plate of claim 1, wherein the multi-well plate is manually divisible along the center of the segmented breaking features into a plurality of single columns of multi-well strips.

3. The multi-well plate of claim 1 or claim 2, wherein the multi-well plate is configured such that manually dividing the multi-well plate generates one or more columns of multi-well strips that have no sharp edges.

4. The multi-well plate of any preceding claim, wherein the undercuts are V-Shaped undercuts.

5. The multi-well plate of any preceding claim, wherein a chimney located on the frame interlocks with a lug located on a side of the well.

6. The multi-well plate of any preceding claim, wherein the first material comprises polycarbonate and the second material comprises polypropylene.

7. The multi-well plate of any preceding claim, wherein the wells are directly moulded into the frame utilising form locking connections.

8. The multi-well plate of any preceding claim, wherein the frame further comprises a skirt that wraps all around the perimeter of the plate.

9. The multi-well plate of any of claims 1-7, wherein the frame further comprises a skirt that is located at the four corners of the plate.

10. The multi-well plate of any preceding claim, comprising 24, 48, 96, 384 or 1536 wells.

11. The multi-well plate of claim 10, comprising 96 wells.

12. The multi-well plate of any preceding claim, wherein the plate can be manually divided into one or more columns of 8 tube strips or into segments comprising at least one column of 8-tube strips.

## Patentansprüche

1. Platte mit mehreren Vertiefungen, umfassend:
einen Rahmen aus einem ersten Material, wobei der Rahmen eine ebene obere Oberfläche aufweist, mit einer Anordnung von einer Vielzahl von Löchern, die in einem Spaltenmuster auf der oberen Oberfläche angeordnet sind, wobei der Rahmen ferner eine Anordnung von segmentierten Bruchmerkmalen umfasst, die in einem Spaltenmuster angeordnet sind, das sich mit dem Spaltenmuster der Vielzahl von Löchern abwechselt; und
eine Vielzahl von Vertiefungen aus einem zweiten Material, wobei die Vertiefungen in die Vielzahl von Löchern auf der oberen Oberfläche des Rahmens eingebracht sind,
wobei die Platte mit mehreren Vertiefungen entlang einer Mitte der segmentierten Bruchmerkmale manuell in eine oder mehrere Spalten von Streifen mit mehreren Vertiefungen teilbar ist,
wobei das segmentierte Bruchmerkmal eine Anordnung von blütenblattförmigen Einschnitten, die sich von der oberen Oberfläche des Rahmens durch eine untere Oberfläche des Rahmens erstrecken, und eine Reihe von Hinterschnitten auf einer unteren Oberfläche des Rahmenabschnitts, derart angeordnet, dass sich die Hinterschnitte mit den blütenblattförmigen Einschnitten abwechseln, umfasst.

2. Platte mit mehreren Vertiefungen nach Anspruch 1, wobei die Platte mit mehreren Vertiefungen manuell entlang der Mitte der segmentierten Bruchmerkmale in eine Vielzahl von einzelnen Spalten von Streifen mit mehreren Vertiefungen teilbar ist.

3. Platte mit mehreren Vertiefungen nach Anspruch 1 oder Anspruch 2, wobei die Platte mit mehreren Vertiefungen so konfiguriert ist, dass das manuelle Teilen der Platte mit mehreren Vertiefungen eine oder mehrere Spalten von Streifen mit mehreren Vertiefungen erzeugt, die keine scharfen Kanten aufweisen.

4. Platte mit mehreren Vertiefungen nach einem der vorstehenden Ansprüche, wobei die Hinterschnitte V-förmige Hinterschnitte sind.

5. Platte mit mehreren Vertiefungen nach einem der vorstehenden Ansprüche, wobei ein auf dem Rahmen angeordneter Schacht einen an einer Seite der Vertiefung angeordneten Vorsprung in Eingriff nimmt.

6. Platte mit mehreren Vertiefungen nach einem der vorstehenden Ansprüche, wobei das erste Material Polycarbonat umfasst und das zweite Material Polypropylen umfasst.

7. Platte mit mehreren Vertiefungen nach einem der vorstehenden Ansprüche, wobei die Vertiefungen unter Verwendung von Formverriegelungsverbindungen direkt in den Rahmen eingegossen sind.

8. Platte mit mehreren Vertiefungen nach einem der vorstehenden Ansprüche, wobei der Rahmen ferner eine Schürze umfasst, die sich um den Umfang der Platte wickelt.

9. Platte mit mehreren Vertiefungen nach einem der Ansprüche 1 bis 7, wobei der Rahmen ferner eine Schürze umfasst, die sich an den vier Ecken der Platte befindet.

10. Platte mit mehreren Vertiefungen nach einem der vorstehenden Ansprüche, 24, 48, 96, 384 oder 1536 Vertiefungen umfassend.

11. Platte mit mehreren Vertiefungen nach Anspruch 10, 96 Vertiefungen umfassend.

12. Platte mit mehreren Vertiefungen nach einem der vorstehenden Ansprüche, wobei die Platte manuell in eine oder mehrere Spalten von 8-Röhrchenstreifen oder in Segmente, die mindestens eine Spalte von 8-Röhrchenstreifen umfassen, geteilt werden kann.

## Revendications

1. Plaque multipuits comprenant :
un cadre constitué d'un premier matériau, dans laquelle le cadre a une surface supérieure plane ayant un ensemble d'une pluralité de trous agencés selon un motif en colonne sur la surface supérieure, le cadre comprenant en outre un ensemble de caractéristiques de rupture segmentées agencées selon un motif en colonne alternant avec le motif en colonne de la pluralité de trous ; et
une pluralité de puits constitués d'un second matériau, dans laquelle les puits sont placés dans la pluralité de trous sur la surface supérieure du cadre,
dans laquelle la plaque multipuits est divisible manuellement le long d'un centre des caractéristiques de rupture segmentées en une ou plusieurs colonnes de bandes multipuits,
dans laquelle la caractéristique de rupture segmentée comprend un ensemble de coupes en forme de pétales qui s'étendent à partir de la surface supérieure du cadre à travers une surface inférieure du cadre et une série de contre-dépouilles sur une surface inférieure de la partie de cadre, agencées de telle sorte que les contre-dépouilles alternent avec les coupes en forme de pétales.

2. Plaque multipuits selon la revendication 1, dans laquelle la plaque multipuits est divisible manuellement le long du centre des caractéristiques de rupture segmentées en une pluralité de colonnes uniques de bandes multipuits.

3. Plaque multipuits selon la revendication 1 ou la revendication 2, dans laquelle la plaque multipuits est conçue de telle sorte que la division manuelle de la plaque multipuits génère une ou plusieurs colonnes de bandes multipuits qui n'ont pas de bords tranchants.

4. Plaque multipuits selon l'une quelconque revendication précédente, dans laquelle les contre-dépouilles sont des contre-dépouilles en forme de V.

5. Plaque multipuits selon l'une quelconque revendication précédente, dans laquelle une cheminée située sur le cadre se verrouille avec un ergot situé sur un côté du puits.

6. Plaque multipuits selon l'une quelconque revendication précédente, dans laquelle le premier matériau comprend du polycarbonate et le second matériau comprend du polypropylène.

7. Plaque multipuits selon l'une quelconque revendication précédente, dans laquelle les puits sont directement moulés dans le cadre à l'aide de liaisons de verrouillage de forme.

8. Plaque multipuits selon l'une quelconque revendication précédente, dans laquelle le cadre comprend en outre une jupe qui s'enroule tout autour du périmètre de la plaque.

9. Plaque multipuits selon l'une quelconque des revendications 1 à 7, dans laquelle le cadre comprend en outre une jupe qui est située aux quatre coins de la plaque.

10. Plaque multipuits selon l'une quelconque revendication précédente, comprenant 24, 48, 96, 384 ou 1536 puits.

11. Plaque multipuits selon la revendication 10, comprenant 96 puits.

12. Plaque multipuits selon l'une quelconque revendication précédente, dans laquelle la plaque peut être divisée manuellement en une ou plusieurs colonnes de bandes de 8 tubes ou en segments comprenant au moins une colonne de bandes de 8 tubes.
